(19) Europäisches Patentamt
European Patent Office
Office européen des brevets

(11) Numéro de publication : 0 302 980 B1

(12) # FASCICULE DE BREVET EUROPEEN

(45) Date de publication du fascicule du brevet : **13.03.91 Bulletin 91/11**

(51) Int. Cl.$^5$ : **C07D 405/04**, C07D 405/14, C07D 409/14, A61K 31/445

(21) Numéro de dépôt : **87401799.9**

(22) Date de dépôt : **03.08.87**

(54) **1,4-Dihydropyridines, leur procédé de préparation et leur application comme médicaments.**

(43) Date de publication de la demande :
**15.02.89 Bulletin 89/07**

(45) Mention de la délivrance du brevet :
**13.03.91 Bulletin 91/11**

(84) Etats contractants désignés :
**AT DE ES FR GB GR IT NL SE**

(56) Documents cités :
**EP-A- 0 158 211**
**EP-A- 0 174 131**
**The Merk Index Manual Data Report (1986)**

(73) Titulaire : **LABORATORIOS DELAGRANGE S.A.**
**Avenida de la Industria, 17 Apartado 101**
**E-28100 Alcobendas (Madrid) (ES)**

(72) Inventeur : **Fernandez Torija, Carlos**
**C/Venezuela, 6**
**Coslada (Madrid) (ES)**
Inventeur : **Galiano Ramos, Joaquin Alvaro**
**Tebas No. 23**
**Las Rozas (Madrid) (ES)**

(74) Mandataire : **Glawe, Delfs, Moll & Partner**
**Patentanwälte**
**Postfach 26 01 62 Liebherrstrasse 20**
**W-8000 München 26 (DE)**

**EP 0 302 980 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux composés aux propriétés antagonistes du calcium, particulièrement puissantes. Ils sont utiles dans le traitement de l'angine de poitrine, de l'attaque cardiaque, de l'hypertension et autres problèmes cardiovasculaires.

Les composés selon l'invention sont chimiquement apparentés aux bloquants calciques du groupe de la 1,4-dihydropyridine tels que l'Oxodipine (I), la Nifédipine (II) et la Nicardipine (IIa) et présentent des effets relaxants sur les muscles cardiaque et vasculaires.

L'Oxodipine synthétisée par l'un des auteurs de la présente invention et surtout recommandée pour traiter une hypertension essentielle d'intensité légère à modérée, présentait déjà une amélioration par rapport à la Nifédipine, dans le sens d'une meilleure stabilité et d'un effet plus durable.

(I) 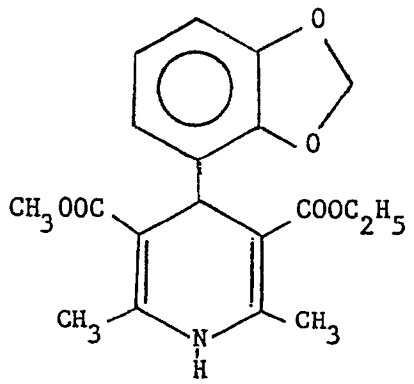

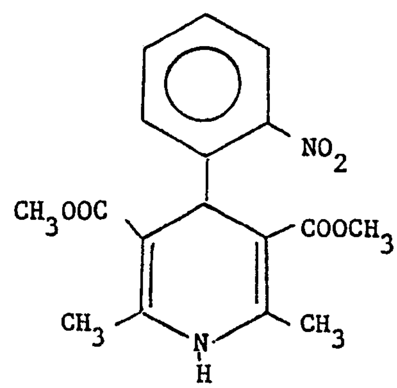 (II)

Oxodipine

Nifédipine

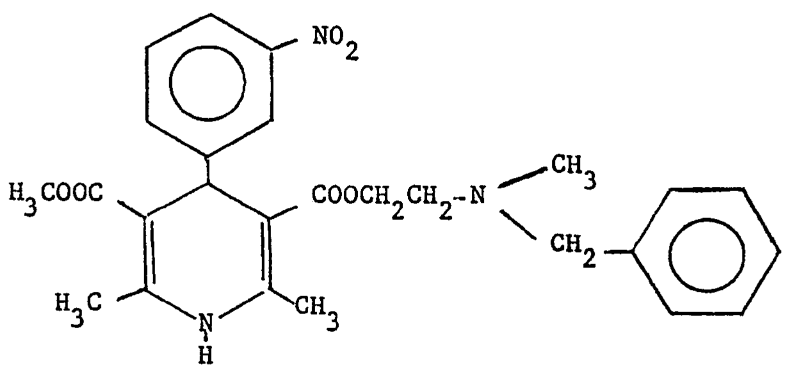 (IIa)

Nicardipine

Les composés de la présente invention sont de nouvelles 1,4-dihydropyridines, douées d'une très haute activité vasodilatatrice et donc de propriétés antiangineuse et antihypertensive. L'invention concerne les composés, de formule générale (III) :

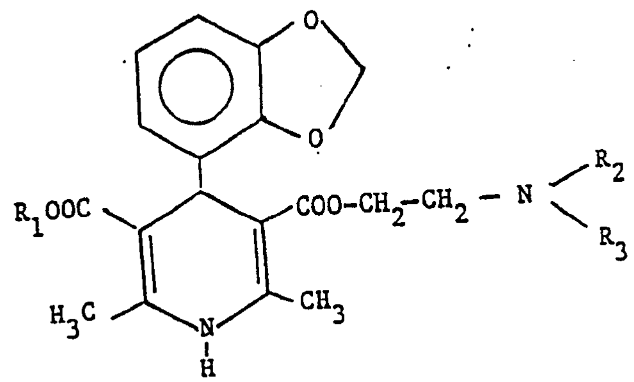

(III)

dans laquelle :

- $R_1$ représente un groupe méthyle, éthyle, propyle, isopropyle, butyle ou isobutyle,
- $R_2$ représente un radical méthyle, formyle ou éthyle,
- $R_3$ représente un atome d'hydrogène ou un groupe 2-, 3- ou 4-picolyle, ou 4-fluorobenzyle,

ainsi que les composés décrits dans les exemples II, III, IX, X et XII.

Ces composés se caractérisent, comme l'Oxodipine, par la présence d'un radical 4-(2',3'-Méthylènedioxy) phényle.

La présence d'un carbone asymétrique (C4) indique la possibilité de 2 énantiomères. La présence d'un groupe amino permet l'existence de sels tels que chlorhydrate, sulfate ou autres, à partir d'acides organiques ou minéraux, pharmaceutiquement acceptables.

La présente invention concerne également un procédé de fabrication de ces composés, consistant à traiter le 2,3-méthylènedioxybenzaldéhyde (IV) :

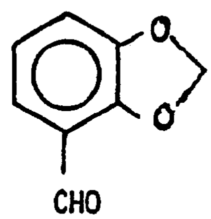

(IV)

par un ester acétoacétique de formule (V) ou (VIII) :

$CH_3-COCH_2-COOR_1$ (V)

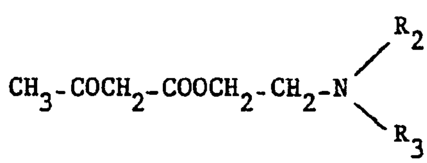

(VIII)

où $R_1$, $R_2$, $R_3$ ont la même signification que dans la formule (III), puis à traiter l'ester α-acétyl β-(2,3-méthylènedioxyphényl)acrylique résultant, de formule (VI) ou (IX) :

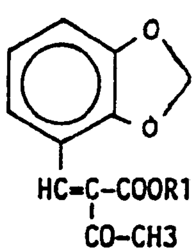

(VI)

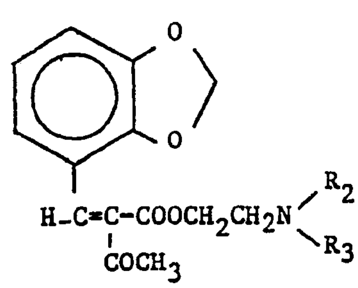

(IX)

où $R_1$, $R_2$, $R_3$ conservent les mêmes significations, par un ester 3-amino crotonique de formule (VII) ou (X) :

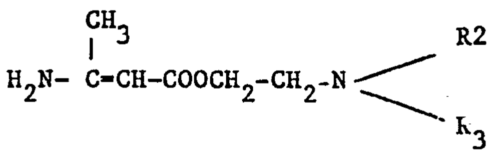 (VII) 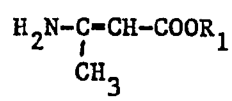 (X)

où $R_1$, $R_2$ $R_3$ ont la même signification que dans la formule (III).

Les esters acétoacétiques sont obtenus par traitement du dicétène, avec l'alcool substitué approprié.

La présente invention comprend les préparations pharmaceutiquement acceptables de ces composés, pouvant être administrées oralement ou par voies rectale, nasale, sublinguale ou parentérale. Ces préparations consistent en un mélange du principe actif, éventuellement sous la forme d'un sel pharmaceutiquement acceptable, avec un véhicule qui peut être solide, semi-solide, liquide ou sous forme de capsules ingérables, formant ainsi un autre aspect de l'invention.

Généralement, le principe actif constitue 0,1% à 99% en poids, de la préparation, par exemple, 0,5 à 10% pour les préparations injectables, et entre 10 et 80% pour les préparations à usage oral.

Dans le cas d'une préparation pharmaceutique contenant un composé selon l'invention, sous la forme de dosages unitaires pour application orale, le principe actif peut être mélangé à un solide pulvérulent tel que lactose, saccharose, sorbitol, ou encore de l'amidon de blé, amylopectine, agar, ou un dérivé de cellulose, polyvinylpyrrolidone, ou gélatine, et peut aussi comprendre des lubrifiants tels que du stéarate de magnésium, des cires de polyéthylèneglycol (Carbowax®), puis être mis sous forme de comprimés ou de noyaux pour pilules.

Si l'on choisit les pilules, les noyaux peuvent être enrobés, par exemple avec une solution concentrée de sucre qui peut contenir de la gomme d'acacia, du talc, avec ou sans oxyde de titane, ou bien avec un agent filmogène dissous dans un solvant organique volatil. On peut ajouter des colorants, par exemple pour distinguer les différents dosages de substance active.

Pour préparer des capsules molles, le principe actif peut être dissous dans une huile appropriée telle que l'huile d'olive, de sésame, ou d'arachide. Les capsules dures peuvent contenir des granulés du principe actif, faits avec un véhicule solide pulvérent tel que le lactose, le saccharose, l'amidon, les dérivés cellulosiques, la polyvinylpyrrolidone, ou la gélatine, avec ou sans lubrifiant tel que stéarate de magnésium, ou acide stéarique.

On peut aussi préparer des formes-retard, ou a diffusion différée, à l'aide d'excipients appropriés. On peut employer différents procédés pour contrôler la disponibilité : micro-granules ou particules enrobées, noyaux à couches successives ou formes légèrement solubles.

On peut enfin préparer des formes pharmaceutiques liquides pour l'administration orale : élixirs, sirops, ou suspensions. Par exemple, on peut utiliser une solution contenant 0,1 à 10% en poids de principe actif, avec du sucre dans un mélange alcool-eau-glycérine ou propylèneglycol, aromatisé ou non, contenant de la saccharine, de la carboxyméthylcellulose, ou de la pectine comme agent dispersant.

Pour l'administration parentérale, on peut préparer des solutions aqueuses contenant 0,1 à 0,5% des principes actifs selon l'invention, capables de former des sels avec des acides tels que l'acide chlorhydrique, phosphorique, tartrique, ou d'autres acides organiques ou minéraux. Des dosages unitaires de la solution, éventuellement stabilisée ou tamponnée, peuvent avantageusement être présentés dans des ampoules ou des flacons.

La posologie peut varier, et dépend de plusieurs facteurs, tels que l'état du patient. Les doses préférables vont de 10 à 50 mg, administrées 1 à 3 fois par jour. Par voie parentérale, la dose administrée est généralement de 1 à 10 mg.

Quelques exemples de préparation des composés de l'invention vont être donnés afin de l'illustrer, sans toutefois la limiter à ceux-ci.

## Exemple I

### 1,4-dihydro 2,6-diméthyl 4-(2',3'-méthylènedioxyphényl)-3-carboxylate de méthyle 5-carboxylate de 2'-(N-méthylamino)éthyle pyridine :

Dans un autoclave de 100 ml, on verse 0,5 g (0,97 m.mole) de chlorhydrate de 1,4-dihydro 2,6-diméthyl 4-(2',3'-méthylènedioxyphényl) 3-carboxylate de méthyle 5-carboxylate de 2'-(N-méthyl N-benzylamino)éthyle pyridine (préparé selon le brevet Espagnol N° 531.033), 15 ml d'éthanol séché, et 110 mg de charbon palladié à 10%.

Le mélange réactionnel est maintenu sous pression de 10 psi d'hydrogène pendant 20 minutes, avec agitation.

Puis le mélange est filtré sur papier Whatman pour évacuer le catalyseur, et le solvant est évaporé sous vide. On obtient une huile qu'on triture avec de l'éther di-isopropylique. Après filtration, on recueille 0,42 g d'une

poudre cristalline.

En CCM, le chlorhydrate donne une tache simple (chloroforme-acétone 5-1 en volume).

Spectre IR (K Br)

3600-2600 $cm^{-1}$ (plusieurs bandes, chlorhydrate d'amine).
1700-1680 $cm^{-1}$ (C=O, groupe ester).

Le chlorhydrate obtenu est mis en suspension dans 5 ml d'une solution 2 N de soude, et le mélange est agité pendant 10 minutes. Après extraction par 2 fois 10 ml de chloroforme, la couche organique est décantée, séchée sur sulfate de sodium anhydre, et le solvant est évaporé sous vide. Le résidu est cristallisé dans de l'éther di-isopropylique.

On obtient 0,36 g (83%) d'un solide blanc cristallisé.

PF = 144-146°C

spectre IR (K Br)

3310 $cm^{-1}$ (N-H, du cycle dihydropyridine)
3200 $cm^{-1}$ (N-H, de la chaîne latérale N-méthylaminoéthyle)
1715-1700 $cm^{-1}$ (C=O, groupes ester)
1660 $cm^{-1}$ (C=C de la dihydropyridine)

Exemple II

1,4-dihydro 2,6-diméthyl 4-(2',3'-méthylènedioxyphényl) 3-carboxylate de méthyle 5- carboxylate de 2'-(N-formyl N-méthylamino)éthyle pyridine.

a) 2-(N-formyl N-méthylamino)éthanol :

Dans un ballon à fond rond contenant 5,2 ml (66 m.mole) de 2-(N-méthylamino)éthanol, on ajoute sous agitation 6 ml (98 m.mole) de formiate de méthyle, en 2 fractions, en maintenant la température du mélange au-dessous de 50°C, car la réaction est exothermique.

Après 40 minutes d'agitation, on retire sous vide l'excès de formiate de méthyle et le méthanol produit au cours de la réaction. On obtient 5,8 g de produit sous la forme d'un liquide incolore qu'on utilise tel que pour l'étape suivante (rendement de 100%).

Spectre IR (K Br)

3600-3100 $cm^{-1}$ (OH avec liaisons hydrogène)
1650 $cm^{-1}$ (C=O formamide)

b) acétylacétate de 2-(N-formyl N-méthylamino)éthyle :

Dans un ballon à fond rond équipé d'un réfrigérant et d'une ampoule à brome, on chauffe vers 75-85°C 19,27 g (187 m.mole) de 2-(N-formyl N-méthylamino)éthanol et 65 mg (0,8 m.mole) d'acétate de sodium fraîchement fondu. 15,61 g (187 m.mole) de cétène dimère sont ensuite ajoutés goutte à goutte, en maintenant la température dans l'intervalle 75-85°C. Lorsque l'addition est terminée, le mélange est agité pendant encore 1 heure à 75-85°C.

On obtient 34,41 g (100%) d'une huile brunâtre, qu'on utilise sans distillation pour l'étape suivante.

Spectre IR (K Br)

1740 et 1710 $cm^{-1}$ (C=O groupes ester et cétone)
1660 $cm^{-1}$ (C=O formamide)

c) acrylate de 2-(N-formyl N-méthylamino)éthyle, α-acétyl β-(2',3'-méthylènedioxyphényl) :

Dans un ballon à fond rond, relié à un séparateur Dean-Stark contenant du benzène sec, on dissout 2,5 g (16,6 m.mole) de 2,3-méthylènedioxybenzaldéhyde, et 3,2 g d'acétylacétate de 2-(N-formyl N-méthylamino)éthyle dans 7 ml de benzène sec. Le mélange est chauffé jusqu'à dissolution et on ajoute alors 0,06 ml de pipéridine et 0,2 ml d'acide acétique glacial. La solution résultante est portée à reflux pendant 1 heure jusqu'à ce qu'il ne s'élimine plus d'eau. Après refroidissement, le mélange est dilué avec 30 ml de benzène et lavé avec 2 fois 30 ml d'acide chlohydrique à 5%, puis 2 fois 30 ml de bicarbonate de soude à 5% et finalement par 30 ml d'eau. La couche organique est décantée, séchée sur sulfate de magnésium anhydre et évaporée sous vide. L'huile résultante est lavée par 15 ml d'éther pour enlever le benzaldéhyde qui n'a pas réagi. Après évaporation du solvant résiduel, 2,7 g (51%) d'huile brune et visqueuse est obtenue. On l'utilise telle que pour l'étape suivante.

Spectre IR (K Br)

1720 $cm^{-1}$ (C=O ester)
1680-1640 $cm^{-1}$ (C=C et C=O des groupes cétone et formamide)

d) 1,4-dihydro 2,6-diméthyl 4-(2',3'-méthylènedioxyphényl) 3-carboxylate de méthyle 5-carboxylate de 2'-(N-formyl N-méthylamino)éthyle pyridine

2,7 g (8,4 m.mole) d'acrylate de 2-(N-formyl N-méthylamino)éthyle, α-acétyl β-(2',3'-méthylènedioxyphényl) et 0,96 g (8,4 m.mole) de 3-aminocrotonate de méthyle sont dissous dans 10 ml d'isopropanol.

Le mélange est maintenu deux jours à 37°C dans une étuve et le solvant est ensuite évaporé sous vide. L'huile résiduelle est traitée par un mélange de n-héxane/éther di-isopropylique, pour obtenir une huile claire qui cristallise. On recueille le solide par filtration et on obtient 2,9 g (83%) de cristaux blanc-jaune.

PF = 142-144°C

Spectre IR (K Br)

3260 $cm^{-1}$ (N-H du cycle pyridine)
1700-1690 $cm^{-1}$ (C=O groupes ester)
1660-1640 $cm^{-1}$ (C=O formamide et C=C de la dihydropyridine)

Exemple III

1,4-dihydro 2,6-diméthyl 4-(2',3'-méthylènedioxyphényl) 3-carboxylate de méthyle 5-carboxylate de 2'(N-formyl N-benzylamino)éthyle pyridine.

a) 2-(N-formyl N-benzylamino)éthanol :

10 g (66 m.mole) de 2-(N-benzylamino)éthanol sont mélangés avec 7,5 ml (121 m.mole) de formiate de méthyle. Le mélange est agité pendant 3 heures à 50-60°C et l'excès de formiate de méthyle est éliminé sous vide avec le méthanol produit ; on obtient 11,9 g de liquide incolore qui est utilisé sans distillation pour l'étape suivante. (rendement 100%)

Spectre IR (K Br)

3600-3200 $cm^{-1}$ (OH avec liaisons hydrogène)
1680-1650 $cm^{-1}$ (C=O formamide)

b) acétylacétate de 2-(N-formyl N-benzylamino)éthyle :

Ce composé est préparé par réaction de 2-(N-formyl N-benzylamino)éthanol et de cétène dimère comme décrit dans l'exemple IIb. Le produit obtenu est utilisé tel que, pour l'étape suivante. (rendement 100%)

Spectre IR (K Br)

1760 et 1730 $cm^{-1}$ (C=O des goupes ester et cétone)
1680-1660 $cm^{-1}$ (C=O formamide)

c) acrylate de 2-(N-formyl N-benzylamino)éthyle α-acétyl β-(2',3'-méthylènedioxyphényl) :

Ce composé est préparé par réaction de l'acétylacétate de 2-(N-formyl N-benzylamino)éthyle et de 2,3-méthylènedioxybenzaldéhyde, selon le procédé décrit dans l'exemple IIc.

Le produit brut est traité par un mélange éther éthylique/éther de pétrole pour éliminer le benzaldéhyde de départ résiduel, et on obtient une huile orange. (rendement 55%)

Spectre IR (K Br)

1740 $cm^{-1}$ (C=O ester)
1710-1670 $cm^{-1}$ (C=C, C=O des groupes cétone et formamide)

d) 1,4-dihydro 2,6-diméthyl 4-(2',3'-méthylènedioxyphényl) 3-carboxylate de méthyle 5-carboxylate de 2'-(N-formyl N-benzylamino)éthyle pyridine

Ce composé est préparé par réaction de l'acrylate de 2- (N-formyl N-benzylamino)éthyle α-acétyl β-(2',3'-méthylènedioxyphényl) et de 3-aminocrotonate de méthyle, selon le procédé décrit dans l'exemple IId.
Le produit est recristallisé dans l'éthanol, et donne un solide cristallisé légèrement jaune. (rendement 76%)

PF = 165-167°C.

Spectre IR (K Br)

3300 $cm^{-1}$ (N-H de la dihydropyridine)
1715-1700 $cm^{-1}$ (C=O des groupes ester)
1680-1650 $cm^{-1}$ (C=O formamide et C=C du cycle dihydropyridine)

Exemple IV

1,4-dihydro 2,6-diméthyl 4-(2',3'-méthylènedioxyphényl) 3-carboxylate de méthyle 5-carboxylate de 2-(N-méthyl N-(2-picolyl)amino)éthyle pyridine :

a) 2-(N-méthyl N-(2-picolyl)amino)éthanol) :

Dans un flacon à fond rond, sous atmosphère d'azote, on verse 57,6 ml (0,72 mole) de 2-(N-méthylamino)éthanol, 30 ml d'eau et 34,02 g (0,405 mole) de bicarbonate de soude. Le mélange est agité vigoureusement et chauffé à 90-95°C. 30 g de chlorure de 2-picolyle (chlorhydrate) sont ajoutés en 3 fois, sous forme de 3 solutions fraîchement préparées de 10 g dans 25 ml d'eau, l'addition étant répartie sur 1 heure. Après addition totale, on agite encore pendant 3 heures à 90-95°C. L'eau est alors éliminée sous vide, l'huile résiduelle est laissée refroidir, puis filtrée pour séparer les sels minéraux précipités.
Le filtrat est dilué avec 400 ml de chloroforme, et la solution résultante est lavée par 2 fois 400 ml de bicarbonate de soude à 5%, puis séchée sur carbonate de potassium anhydre. Le solvant est éliminé sous pression réduite, donnant 23,19 g (76%) d'une huile claire que l'on utilise telle que, sans purification, pour l'étape suivante.

Spectre IR (K Br)

3600-3200 $cm^{-1}$ (OH avec liaisons hydrogène)

b) acétylacétate de 2-(N-méthyl N-(2-picolyl)amino)éthyle :

Ce composé est préparé par réaction de 2-(N-méthyl N-(2-picolyl)amino)éthanol et de cétène dimère selon le procédé décrit à l'exemple IIb ; on obtient un liquide noir présentant 3 taches en CCM. (chloroforme/acétone,

5/1, lampe UV à 245 n.m)
Le produit brut est instable lorsqu'on le chauffe au point d'ébullition sous 8-9 mm de mercure, et donc on l'utilise sans le purifier pour l'étape suivante.

Spectre IR (K Br)

1740 $cm^{-1}$ (ester)
1 710 $cm^{-1}$ (C=O groupe cétonique)

c) 3-aminocrotonate de 2-(N-méthyl N-(2-picolyl)amino)éthyle :

Dans un ballon à fond rond, équipé d'un thermomètre et d'une arrivée de gaz, on dissout 5 g d'acétylacétate de 2-(N-méthyl N-(2-picolyl)amino)éthyle, dans 7 ml de méthanol. La solution est refroidie par un mélange glace-eau, et un courant d'ammoniac est envoyé à travers, jusqu'à saturation du mélange réactionnel (environ 2 heures), la température étant maintenue au-dessous de 25°C. Le solvant est ensuite évaporé sous vide, donnant une huile noire qu'on purifie par ébullition dans de l'éther isopropylique, puis séparation de la fraction insoluble, pour obtenir 4,1 g d'huile orange.

Spectre IR (K Br)

3400 et 3300 $cm^{-1}$ ($NH_2$)
1660 $cm^{-1}$ (C=O ester conjugué)
1620-1610 $cm^{-1}$ (C=C)

d) dichlorhydrate de 1,4-dihydro 2,6-diméthyl 4-(2',3'-méthylènedioxyphényl) 3-carboxylate de méthyle 5-carboxylate de 2-(N-méthyl N-(2-picolyl)amino)éthyle pyridine :

2 g (8 m.mole) d'acrylate de méthyle α-acétyl β-(2,3-méthylènedioxyphényl) et 2,05 g (8,7 m.mole) de 3-aminocrotonate de 2-(N-méthyl N-(2-picolyl)amino)éthyle sont dissous dans 15 ml d'isopropanol. La solution est laissée au repos pendant 2 hours à 37°C et le solvant est éliminé sous vide. L'huile résiduelle est traitée par de l'éther isopropylique, à chaud, et donne une fraction insoluble qui est recueillie par décantation. L'éther restant est éliminé sous vide, et le solide est dissous dans 20 ml d'isopropanol. Après refroidissement par un mélange glace-eau, 1,3 ml d'acide chlorhydrique (6,4 N) dans l'isopropanol est ajouté goutte à goutte. Le solide qui précipite est laissé cristalliser pendant 3 jours et on le sépare par filtration. Après cristallisation dans l'isopropanol, on obtient 1,7 g (39%) de dichlorhydrate.

A l'analyse RMN, un peu d'isopropanol apparaît, provoquant la solvatation des cristaux du composé.

PF = 161-164°C, avec décomposition.

Spectre IR (K Br)

3700-2500 $cm^{-1}$ (plusieurs bandes, amine et pyridine sous forme de chlorhydrates)
1710 et 1690 $cm^{-1}$ (C=O ester)

Exemple V

1,4-dihydro 2,6-diméthyl 4-(2',3'-méthylènedioxyphényl) 3-carboxylate de méthyle 5-carboxylate de 2-(N-méthyl N-(4-picolyl)amino)éthyle pyridine.

a) 2-(N-méthyl N-(4-picolyl)amino)éthanol :

Ce composé est préparé à partir du chlorure de 4-picolyle et du 2-(N-méthylamino)éthanol, selon le procédé décrit dans l'exemple IVa ; on obtient une huile qu'on utilise telle que, sans purification, pour l'étape suivante. (rendement 79%)

Spectre IR (K Br)

3600-3100 $cm^{-1}$ (OH avec liaisons hydrogène)

b) acétylacétate de 2-(N-méthyl N-(4-picolyl)amino)éthyle :

Ce composé est préparé par réaction de 2-(N-méthyl N-(4-picolyl)amino)éthanol et de cétène dimère comme il est décrit à l'exemple IIb. Le produit est utilisé tel que pour l'étape suivante, sans purification.

Spectre IR (K Br)

1760 $cm^{-1}$ (C=O ester)
1735 $cm^{-1}$ (C=O groupe cétonique)

c) 3-aminocrononate de 2-(N-méthyl N-(4-picolyl)amino)éthyle :

Ce composé est préparé à partir de l'acétylacétate correspondant suivant le procédé décrit à l'exemple IVc.

Spectre IR (K Br)

3400 et 3300 $cm^{-1}$ ($NH_2$)
1660 $cm^{-1}$ (C=O ester conjugué)
1620-1610 $cm^{-1}$ (C=C)

d) 1,4-dihydro 2,6-diméthyl 4-(2',3'-méthylènedioxyphényl) 3-carboxylate de méthyle 5-carboxylate de 2-(N-méthyl N-(4-picolyl)amino)éthyle pyridine :

Ce composé est obtenu par réaction d'acrylate de méthyle α-acétyl β-(2',3'-méthylènedioxyphényl), et de 3-aminocrotonate de 2-(N-méthyl N-(4-picolyl)amino)éthyle selon le procédé décrit à l'exemple IVd. Le produit ne peut être obtenu à l'état cristallisé, car il est hygroscopique. On l'obtient sous forme solide amorphe et hygroscopique par suspension dans de l'acétate d'éthyle anhydre, puis évaporation du solvant sous vide. (rendement 41%)

Spectre IR (K Br)

3600-2400 $cm^{-1}$ (plusieurs bandes, chlorhydrates de l'amine et de la pyridine)
1700-1680 $cm^{-1}$ (C=O ester)

Exemple VI

1,4-dihydro 2,6-diméthyl 4-(2',3'-méthylènedioxyphényl) 3-carboxylate de méthyle 5-carboxylate de 2-(N-méthyl N-(3-picolyl)amino)éthyle pyridine.

a) 2-(N-méthyl N-(3-picolyl)amino)éthanol :

Ce composé est préparé à partir du chlorure de 3-picolyle et du 2-(N-méthylamino)éthanol, selon le procédé décrit à l'exemple IVa ; on obtient une huile qui est utilisée directement pour l'étape suivante, sans purification. (rendement 77%)

Spectre IR (K Br)

3600-3100 $cm^{-1}$ (OH avec liaison hydrogène)

b) acétylacétate de 2-(N-méthyl N-(3-picolyl)amino)éthyle :

Ce composé est préparé par réaction de 2-(N-méthyl N-(3-picolyl)amino)éthanol et de cétène dimère, comme décrit dans l'exemple IIb. Le produit est utilisé tel que pour l'étape suivante, sans purification.

Spectre IR (K Br)

1760-1750 $cm^{-1}$ (C=O ester)
1735-1725 $cm^{-1}$ (C=O groupe cétone)

c) 3-aminocrotonate de 2-(N-méthyl N-(3-picolyl)amino)éthyle :

Ce composé est préparé à partir de l'acétylacétate correspondant selon le procédé décrit à l'exemple IVc.

Spectre IR (K Br)

3450 et 3350 $cm^{-1}$ ($NH_2$)
1680-1670 $cm^{-1}$ (C=O ester conjugué)

d) dichlorhydrate de 1,4-dihydro 2,6-diméthyl 4-(2',3'-méthylènedioxyphényl) 3-carboxylate de méthyle 5-carboxylate de 2-(N-méthyl N-(3-picolyl) amino)éthyle pyridine :

Ce composé est obtenu par réaction d'acrylate de méthyle α-acétyl β-(2',3'-méthylènedioxyphényl) et de 3-aminocrotonate de 2-(N-méthyl N-(3-picolyl)amino)éthyle, selon le procédé décrit à l'exemple IVd. En raison de son caractère hygroscopique, le produit ne peut être obtenu sous forme cristalline. On peut l'obtenir sous la forme d'un solide amorphe hygroscopique, par l'ébullition dans de l'éther di-isopropylique et filtration. Puis on le dissout dans l'eau, on lave au chloroforme et on alcalinise à pH 10 avec de la soude 5 N. Après extraction au chloroforme, la couche organique est séchée sur sulfate de magnésium anhydre, et le solvant est évaporé sous vide. On obtient une huile qui cristallise dans l'éther di-isopropylique. La base est une poudre cristalline blanc-jaune, fondant à 139-141°C. (rendement 43%)

Spectre IR (K Br) : de la base.

1710 et 1700 $cm^{-1}$ (C=O ester)
1655 et 1635 $cm^{-1}$ (C=C)

Exemple VII

1,4-dihydro 2,6-diméthyl 4-(2',3'-méthylènedioxyphényl) 3-carboxylate de méthyle 5-carboxylate de 2-(N-méthyl N-(4'-fluorobenzyl)amino)éthyle pyridine.

a) 2-(N-méthyl N-(4'-fluorobenzyl)amino)éthanol :

Dans un ballon pourvu d'un agitateur magnétique, on verse 62,19 g (0,828 mole) de 2-(N-méthylamino)éthanol, 36 ml d'eau et 21,73 g (0,258 mole) de bicarbonate de soude. Le mélange est agité vigoureusement et chauffé à 90-95°C. 30 g de chlorure de 4-fluorobenzyle sont ajoutés goutte à goutte, puis le mélange est maintenu pendant 2 heures à 90-95°C. Après refroidissement à température ambiante, 50 ml d'eau sont ajoutés et on filtre les sels précipités. Le filtrat est extrait par 2 fois 200 ml de chloroforme, et la couche chloroformique est lavée 2 fois par 50 ml d'eau, puis séchée sur sulfate de magnésium anhydre. Le solvant est évaporé sous vide. On obtient 28,2 g (74,4%) d'une huile claire. Le produit est assez pur pour être utilisé tel que dans l'étape suivante.

Spectre IR (K Br)

3600-3200 $cm^{-1}$ (OH)
1620 $cm^{-1}$ (C=C aromatique)
1540 $cm^{-1}$ (aromatique)

b) acétylacétate de 2-(N-méthyl N-(4'-fluorobenzyl)amino)éthyle :

Dans un ballon à fond rond équipé d'un agitateur magnétique, d'un réfrigérant à reflux, d'un thermomètre, et d'une ampoule à brome, on chauffe à 75-85°C un mélange de 18,2 g (99 m.mole) de 2-(N-méthyl N-(4'-fluorobenzyl)amino)éthanol et 36 g d'acétate de sodium anhydre. Lorsque la température est atteinte, 7,67 ml (100

m.mole) de cétène dimère sont ajoutés goutte à goutte, tandis qu'on maintient la température entre 75 et 85°C. L'addition achevée, on maintient le mélange sous agitation, à la même température, pendant une heure. Puis l'excès de cétène est éliminé sous vide. On obtient 26,45 g (100%) d'une huile brune, qu'on utilise telle que pour l'étape suivante.

Spectre IR (K Br)

1750 $cm^{-1}$ (C=O ester)
1720 $cm^{-1}$ (C=O groupe cétonique)

c) 3-aminocrotonate de 2-(N-méthyl N-(4'-fluorobenzyl)amino)éthyle :

Dans un ballon à fond rond équipé d'une arrivée de gaz et d'un thermomètre, on dissout 13,5 g (50 m.mole) d'acétylacétate de 2-(N-méthyl N-(4'-fluorobenzyl)amino)éthyle dans 15 ml de méthanol sec. La solution est refroidie au bain glace-eau et on fait barbotter un courant d'ammoniac sec dans le mélange réactionnel, jusqu'à saturation (généralement atteinte en 2 heures), en maintenant la température au-dessous de 25°C. Le solvant est évaporé sous vide jusqu'à l'obtention d'une huile noire. On ajoute alors 100 ml d'éther de pétrole (PE = 60/80°C) et on porte à ébullition sous vigoureuse agitation, pendant 10 minutes. Le résidu solide est alors éliminé. On évapore le solvant sous vide, et on obtient 12,3 g (rendement 92%) d'huile orange.

Spectre IR (K Br)

3460 et 3350 $cm^{-1}$ ($NH_2$)
1660 $cm^{-1}$ (ester conjugué)
1630 $cm^{-1}$ (C=C)

d) chlorhydrate de 1,4-dihydro 2,6-diméthyl 4-(2',3'-méthylènedioxyphényl) 3-carboxylate de méthyle 5-carboxylate de 2-(N-méthyl N-(4'-fluorobenzyl)amino)éthyle pyridine :

1,5 g (6 m.mole) d'acrylate de méthyle α-acétyl β-(2,3-méthylènedioxyphényl) et 1,59 g (6 m.mole) de 3-aminocrotonate de 2-(N-méthyl N-(4'-fluorobenzyl)amino)éthyle sont dissous dans 7 ml d'isopropanol. Le mélange est laissé au repos 24 heures à 37°C, en étuve. Le solvant est évaporé sous vide et l'huile résiduelle est dissoute dans 60 ml de chloroforme. Après addition de 20 ml d'acide chlorhydrique 6 N, le mélange est agité pendant 10 minutes. La couche organique est décantée, séchée sur sulfate de magnésium anhydre, et le solvant est évaporé sous vide. Le résidu est dissous dans 10 ml d'acétone chaude et cristallise au refroidissement. Le solide est recueilli par filtration, et lavé avec de l'acétone froide. On obtient 1,3 g (40%) du produit sous forme de chlorhydrate.

PF = 205-206°C

Spectre IR (K Br)

3700-2500 $cm^{-1}$ (plusieurs bandes, chlorhydrate de l'amine et N-H du cycle dihydropyridine)
1715 et 1710 $cm^{-1}$ (C=O ester)

Exemple VIII

1,4-dihydro 2,6-diméthyl 4-(2',3'-méthylènedioxyphényl) 3-carboxylate d'éthyle 5-carboxylate de 2-(N-méthyl N(4'-fluorobenzyl)amino)éthyle pyridine.

1 g (3,8 m.mole) d'acrylate d'éthyle α-acétyl β-(2,3-méthylènedioxyphényl) et 1,01 g (3,8 m.mole) de 3-aminocrotonate de 2-(N-méthyl N-(4'-fluorobenzyl)amino)éthyle sont dissous dans 6,5 ml d'isopropanol. La solution est laissée au repos 24 heures à 37°C, en étuve, puis le solvant est évaporé sous vide. L'huile résiduelle est disoute dans 55 ml de chloroforme, et 17 ml d'acide chorhydrique 6 N sont ajoutés. On agite le mélange pendant 10 minutes. La couche organique est décantée et séchée sur sulfate de magnésium anhydre. Après élimination du solvant sous vide, l'huile résiduelle est dissoute dans 8 ml d'acétone chaude et on laisse cristalliser. On obtient 0,95 g (rendement 45,7%) du produit sous forme de chlorhydrate.

PF =221-222°C

Spectre IR (K Br)

3600-2300 $cm^{-1}$ (plusieurs bandes, chlorhydrate de l'amine et N-H du cycle dihydropyridine)
1705-1695 $cm^{-1}$ (doublet, C=O, groupe ester)

Exemple IX

1,4-dihydro 2,6-diméthyl 4-(2',3'-méthylènedioxyphényl) 3-carboxylate de méthyle 5-carboxylate de 2-(N-méthyl N-(2'-thiényl)méthyl amino)éthyle pyridine.

a) 2-(N-méthyl N-(2'-thiényl)méthyl amino)éthanol :

Ce composé est préparé par réaction de 2-chlorométhylthiophène (org. synthèses col. vol. 3 page 197) et de 2-(N-méthylamino)éthanol, selon le procédé décrit à l'exemple VIIa. (rendement 78%)

Le produit est utilisé tel que pour l'étape suivante, sans purification.

Spectre IR (K Br)

3600-3200 $cm^{-1}$ (OH, liaison hydrogène)

b) acétylacétate de 2-(N-méthyl N-(2'-thiényl)méthyl amino)éthyle

Le produit est préparé par réaction de 2-(N-méthyl N-(2'-thiényl)méthyl amino)éthanol et de cétène, selon le procédé décrit à l'exemple VIIb. (rendement 100%). Le produit est utilisé pour l'étape suivante, sans aucune purification.

Spectre IR (K Br)

1760 $cm^{-1}$ (C=O ester)
1730 $cm^{-1}$ (C=O groupe cétone)

c) acrylate de 2-(N-méthyl N-(2'-thiénylméthyl)amino)éthyle α-acétyl β-(2,3-méthylènedioxyphényl) :

Dans un flacon à fond rond, équipé d'un séparateur Dean-Stark contenant du benzène sec, on verse 8,55 g (56,9 m.mole) de 2,3-méthylènedioxybenzaldéhyde et 15 g (58,7 m.mole) d'acétylacétate de 2-(N-méthyl N-(2'-thiénylméthyl)amino)éthyle, dans 35 ml de benzène sec. Le mélange est chauffé jusqu'à complète dissolution, puis on ajoute 0,2 ml de pipéridine et 0,68 ml d'acide acétique glacial. La solution résultante est portée à reflux jusqu'à ce qu'il ne s'élimine plus d'eau (1,5 heure). Le solvant est éliminé sous vide et le résidu est lavé 2 fois par 50 ml d'acide chlorhydrique à 5%. La couche aqueuse est retirée et l'huile résiduelle est dissoute dans 100 ml de chloroforme. La solution chloroformique est lavée avec 2 fois 50 ml de soude à 10%. La couche organique est décantée, séchée sur sulfate de magnésium anhydre, et le solvant est éliminé sous pression réduite. Le résidu huileux est lavé avec un mélange de 30 ml de n-hexane et 3 ml d'éther, pour extraire l'aldéhyde non réagi. Le solvant est retiré, et l'huile est séchée sous vide pour extraire les dernières traces. On obtient 11,5 g (52,6%) d'une huile orange.

Spectre IR (K Br)

1740 $cm^{-1}$ (C=O ester)
1710 $cm^{-1}$ (C=O groupe cétone)
1680 $cm^{-1}$ (C=C)

d) chlorhydrate de 1,4-dihydro 2,6-diméthyl 4-(2',3'-méthylènedioxyphényl) 3-carboxylate de méthyle 5-carboxylate de 2-(N-méthyl N-(2'-thiényl méthyl)amino) éthyle pyridine :

3 g (8,07 m.mole) d'acrylate de 2-(N-méthyl N-(2'-thiényl méthyl)amino)éthyle α-acétyl β-(2,3-méthylènedioxyphényl) et 0,929 g (8,07 m.mole) de 3-aminocrotonate de méthyle, sont dissous dans 10 ml d'isopropanol. La solution est laissée au repos pendant 24 heures à 37°C, puis le solvant est évaporé sous vide. L'huile résiduelle est dissoute dans 100 ml de chloroforme, puis on ajoute 4 fois 30 ml d'acide chlorhydrique 6 N, sous agitation, en 10 minutes. On recueille la couche organique, on la sèche sur sulfate de magnésium anhydre, et on élimine le solvant sous vide. L'huile résiduelle est reprise par 18 ml d'acétone chaude et laissée cristalliser. Les cristaux sont séparés par filtration et lavés à l'acétone froide. On obtient 2,2 g (52%) du produit sous forme de chlorhydrate.

PF = 207-209°C

Spectre IR (K Br)

3600-2300 $cm^{-1}$ (plusieurs bandes, chlorhydrate de l'amine et N-H du cycle dihydropyridine)
1715-1705 $cm^{-1}$ (doublet, C=O des groupes ester)

Exemple X

1,4-dihydro 2,6-diméthyl 4-(2',3'-méthylènedioxyphényl) 3-carboxylate d'éthyle 5-carboxylate de 2-(N-méthyl N-(2'-thiényl méthyl)amino) éthyle pyridine.

Ce composé est préparé à partir d'acrylate de 2-(N-méthyl N-(2'-thiénylméthyl)aminno)éthyle α-acétyl, β-(2,3-méthylènedioxyphényl) et de 3-aminocrotonate d'éthyle, selon le procédé décrit dans l'exemple IXd. (rendement 58,2%). Le produit est obtenu sous forme de chlorhydrate.

PF = 193-195°C.

Spectre IR (K Br)

3600-2300 $cm^{-1}$ (plusieurs bandes, chlorhydrate d'amine et N-H du cycle dihydropyridine)
1715-1705 $cm^{-1}$ (C=O ester)

Exemple XI

1,4-dihydro 2,6-diméthyl 4-(2',3'-méthylènedioxyphényl) 3-carboxylate d'isopropyle 5-carboxylate de 2-(N-méthyl N-(4'-fluorobenzyl)amino) éthyle pyridine.

a) acrylate de 2-(N-méthyl N-(4'-fluorobenzyl)amino)éthyle α-acétyl β-(2,3-méthylènedioxyphényl) :

Le produit est préparé par réaction de 2,3-méthylènedioxybenzaldéhyde et d'acétylacétate de 2-(N-méthyl N-(4'-fluorobenzyl)amino)éthyle selon le procédé décrit dans l'exemple IXc. (rendement 73,8%)

Spectre IR (K Br)

1740 $cm^{-1}$ (C=O ester)
1720 $cm^{-1}$ (C=O groupe cétone)
1680 $cm^{-1}$ (C=C)

b) chlorhydrate de 1,4-dihydro 2,6-diméthyl 4-(2',3'-méthylène dioxyphényl) 3-carboxylate d'isopropyle 5-carboxylate de 2-(N-méthyl N-(4'-fluorobenzyl)amino)éthyle pyridine :

Ce composé est préparé à partir du 3-aminocrotonate d'isopropyle et de l'acrylate de 2-(N-méthyl N-(4'-fluorobenzyl)amino)éthyle α-acétyl β-(2,3-méthylènedioxyphényl), selon le procédé décrit dans l'exemple VIId. Le produit est obtenu sous forme de chlorhydrate, avec un rendement de 36%.

PF = 194-195°C

Spectre IR (K Br)

3600-2400 $cm^{-1}$ (plusieurs bandes, chlorhydrate de l'amine, et N-H du cycle dihydropyridine)
1720 $cm^{-1}$ et 1710 $cm^{-1}$ (C=O groupes ester)

Exemple XII

1,4-dihydro 2,6-diméthyl 4-(2',3'-méthylènedioxyphényl) 3-carboxylate d'isopropyle 5-carboxylate de 2-(N-méthyl N-(2'-thiénylméthyl)amino) éthyle pyridine.

Le composé est préparé par réaction d'acrylate de 2-(N-méthyl N-(2'-thiénylméthyl)amino)éthyle α-acétyl β-(2,3-méthylènedioxyphényl) et de 3-aminocrotonate d'isopropyle, selon le procédé décrit à l'exemple VIId (rendement 48,3%). Le produit est obtenu sous forme de chlorhydrate.

PF = 180-182°C

Spectre IR (K Br)

3600-2300 $cm^{-1}$ (plusieurs bandes, chlorhydrate de l'amine et N-H du cycle dihydropyridine)
1715 $cm^{-1}$ et 1705 $cm^{-1}$ (C=O groupes ester)

Les exemples suivants illustrent la façon dont les composés de l'invention peuvent être incorporés à des compositions pharmaceutiques.

Exemple XIII

Comprimés.

Chaque comprimé contient :

| | |
|---|---|
| Substance active | 10 à 20,0 mg |
| Amidon de maïs | 25,0 mg |
| Lactose | 190,0 mg |
| Gelatine | 1,5 mg |
| Talc | 12,0 mg |
| Stearate de magnésium | 1,5 mg |

Exemple XIV

Tablettes sublinguales.

Chaque tablette contient :

| | |
|---|---|
| Substance active | 10 à 20,0 mg |
| Lactose | 85,0 mg |
| Agar | 5,0 mg |
| Talc | 5,0 mg |

Exemple XV

Capsules Molles.

Chaque capsule contient :

| | |
|---|---|
| Substance active | 10 à 20,0 mg |
| Glycérine | 150,0 mg |
| Polyoxyéthylèneglycol 400 | 50,0 mg |

| | |
|---|---|
| Eau distillée | 150,0 mg |
| Saccharine | 2,0 mg |

Pharmacologie

Les composés de la présente invention ont été testés pour leurs propriétés d'inhibiteurs calciques par des méthodes standard applicables "in vitro". Quelques composés parmi les plus actifs "in vitro" ont également été testés "in vivo".

Méthodes

1) Tests "in vitro" :

Effets inhibiteurs sur les contractions de l'aorte du rat, induites par 80 mM de K Cl.

Les aortes de rats WISTAR (220-250 g) sacrifiés par décapitation sont prélevées et placées dans un bain à organes de 20 ml, à la température de 34°C, et contenant une solution de KREBS composée comme suit (en m.mole par litre) :

NaCl,137 ; K Cl, 2,7 ; Mg $Cl_2$-6 $H_2O$, 1,04 ; $CaCl_2$-2$H_2O$, 0,8 ; $Na_2HPO_4$-$H_2O$, 0,42 ; $NaHCO_3$, 11,9 ; glucose, 5. Cette solution est oxygènée par un mélange de 95% d'$O_2$ et 5% de $CO_2$ (Furchgott et Bhadakron, 1956).

Après une période de stabilisation de 45 minutes sous 2 g de tension, les contractions maximales de l'artère sont induites par l'addition de KCl dans le bain, jusqu'à ce que l'on atteigne la concentration finale de 80 m-.mole/litre. Après que les contractions soient stabilisées, les composés de l'invention, ou l'OXOPIDINE comme témoin de contrôle, sont ajoutés, en laissant une période d'au moins 10 minutes pour stabiliser la relaxation.

Les composés de la présente invention sont dissous dans l'éthanol pour donner des solutions concentrées à environ 1 mg/ml, à partir desquelles on obtient les dilutions utilisées ci-dessus, de $10^{-13}$ à $10^{-17}$ M, par addition de solution saline. Les concentrations inhibitrices 50 (CI.50) sont déterminées par analyse de la droite de regression.

2) Tests "in vivo" :

2)1- activité antihypertensive :

Quelques composés selon l'invention ont été testés pour leur activité antihypertensive sur des rats hypertendus rénaux conscients. La pression sanguine systolique a été mesurée au niveau de la queue de rats éveillés au moyen d'un manchon gonflable et d'un pressomètre digital L.E. 5000 (Letica Instruments, Barcelone, Espagne). Les mesures ont été réalisées avant l'administration i.p de la substance à étudier, puis 30 minutes, 1, 2, 4, et 5 heures après administration. Les animaux ont été maintenus dans des cylindres plastiques préchauffés, pendant les mesures. Les rats présentant une tension inférieure à 160 mm Hg ont été écartés de l'expérience.

Chaque composé a été administré en suspension à 5% (CMC) par injection i.p.

2)2- D L 50 (dose léthale 50) :

La toxicité aiguë de quelques composés de l'invention a été déterminée chez la souris, selon la méthode de LICHFIELD et WILCOXON. Des groupes de 5 souris pesant 20-25 g ont été traités par des doses i.p croissantes des composés à étudier. La mortalité a été enregistrée dans les 7 jours suivant le traitement, et la D L 50 calculée sur cette base.

Résultats

Les composés selon l'invention montrent "in vitro" une très haute activité à l'encontre des contractions induites par le K Cl sur l'aorte du rat.

Les valeurs suivantes ont été obtenues (Cl 50) :

| | |
|---|---|
| Exemple 1 : | Cl 50 = 1,92 ± 0,91×10-6 M |
| Exemple 2 : | Cl 50 = 5,58 ± 1,20×10-7 M |
| Exemple 4 : | Cl 50 = 8,11 ± 1,44×10-8 M |
| Exemple 5 : | Cl 50 = 2,45 ± 1,56×10-7 M |
| Exemple 6 : | Cl 50 = 9,08 ± 3,70×10-9 M |

| | |
|---|---|
| Exemple 3 : | Cl 50 = 5,28 ± 1,08×10-8 M |
| Exemple 7 : | Cl 50 = 2,75 ± 1,00×10-9 M |
| Exemple 8 : | Cl 50 = 4,00 ± 0,70×10-11 M |
| Exemple 9 : | Cl 50 = 1,30 ± 0,57×10-8 M |
| Exemple 10 : | Cl 50 = 9,04 ± 2,18×10-10 M |
| Exemple 11 : | Cl 50 = 4,10 ± 1,52×10-11 M |
| Exemple 12 : | Cl 50 = 2,25 ± 1,33×10-8 M |
| OXOPIDINE : | Cl 50 = 3,10 ± 2,1×10-9 M |

In vivo, les composés des exemples 8 et 11 ont montré une activité antihypertensive prononcée, sur les rats hypertendus rénaux, la dose de 1 mg/kg diminuant respectivement de 23 et 33% la tension élevée d'origine. Les effets du composé de l'exemple 11 ont été observés pendant plus de 6 heures tandis que ceux du composé de l'exemple 8 étaient plus temporaires.

La D L 50 intrapéritonéale de ces deux composés chez la souris est de :

| | |
|---|---|
| Exemple 8 | D L 50 = 70-80 mg/kg |
| Exemple 11 | D L 50 = 30-40 mg/kg |
| OXOPIDINE | D L 50 = 30-40 mg/kg |

## Revendications

### Revendications pour les pays contractants : DE, FR, GB, IT, NL, SE

1. Dérivés de 1,4-dihydropyridine de formule générale (III)

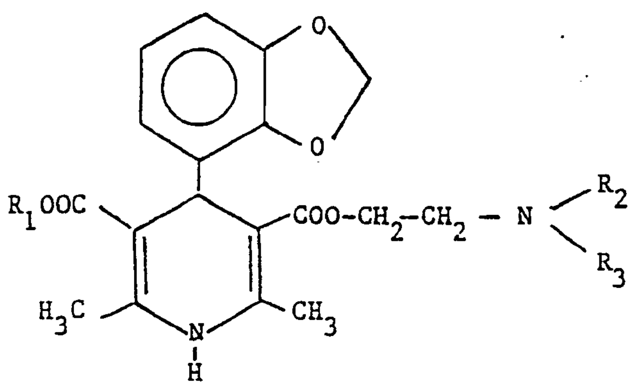

(III)

dans laquelle

– $R_1$ représente un groupe méthyle, éthyle, propyle, isopropyle, butyle ou isobutyle,

– $R_2$ représente un radical méthyle, formyle ou éthyle,

– $R_3$ représente un atome d'hydrogène, ou un groupe 2-, 3-, ou 4-picolyle ou 4-fluorobenzyle,

et leurs sels d'addition pharmaceutiquement acceptables, avec un acide minéral ou organique.

2. Selon la revendication 1, le 1,4-dihydro 2,6-diméthyl 4-(2',3'-éthylènedioxyphényl) 3-carboxylate de méthyle 5-carboxylate de 2-(N-méthylamino)éthyle pyridine.

3. 1,4-dihydro 2,6-diméthyl 4-(2',3'-méthylènedioxyphényl) 3-carboxylate de méthyle 5-carboxylate de 2-(N-formyl N-méthylamino)-éthyle pyridine.

4. 1,4-dihydro 2,6-diméthyl 4-(2',3'-méthylènedioxyphényl) 3-carboxylate de méthyle 5-carboxylate de 2-(N-formyl N-benzylamino)éthyle pyridine.

5. Selon la revendication 1, le 1,4-dihydro 2,6-diméthyl 4-(2',3'-méthylènedioxyphényl) 3-carboxylate de méthyle 5-carboxylate de 2-(N-éthyl N-(2-picolyl)-amino)éthyle pyridine.

6. Selon la revendication 1, le 1,4-dihydro 2,6-diméthyl 4-(2',3'-méthylènedioxyphényl) 3-carboxylate de méthyle 5-carboxylate de 2-(N-méthyl N-(4-picolyl)amino)éthyle pyridine.

7. Selon la revendication 1, le 1,4-dihydro 2,6-diméthyl 4-(2',3'-méthylènedioxyphényl) 3-carboxylate de méthyle 5-carboxylate de 2-(N-méthyl N-(3-picolyl)amino)éthyle pyridine.

8. Selon la revendication 1, le 1,4-dihydro 2,6-diméthyl 4-(2',3'-méthylènedioxyphényl) 3-carboxylate de méthyle 5-carboxylate de 2-(N-méthyl N-(4'-fluorobenzyl)-amino)éthyle pyridine.

9. Selon la revendication 1, le 1,4-dihydro 2,6-diméthyl 4-(2',3'-méthylènedioxyphényl) 3-carboxylate d'éthyle 5-carboxylate de 2-(N-méthyl N-(4'-fluorobenzyl)-amino)éthyle pyridine.

10. 1,4-dihydro 2,6-diméthyl 4-(2',3'-méthylènedioxyphényl) 3-carboxylate de méthyle 5-carboxylate de 2-(N-méthyl N-(2'-thiénylméthyl)amino)éthyle pyridine.

11. 1,4-dihydro 2,6-diméthyl 4-(2',3'-méthylènedioxyphényl) 3-carboxylate d'éthyle 5-carboxylate de 2-(N-méthyl N-(2'-thiénylméthyl)amino)éthyle pyridine.

12. Selon la revendication 1, le 1,4-dihydro 2,6-diméthyl 4-(2',3'-méthylènedioxyphényl) 3-carboxylate d'isopropyle 5-carboxylate de 2-(N-méthyl N-(4'-fluorobenzyl)amino)éthyle pyridine.

13. 1,4-dihydro 2,6-diméthyl 4-(2',3'-méthylènedioxyphényl) 3-carboxylate d'isopropyle 5-carboxylate de 2-(N-méthyl N-(2'-thiénylméthyl)amino)éthyle pyridine.

14. Procédé de préparation des composés de formule (III) selon la revendication 1 qui consiste à traiter le 2, 3-méthylènedioxybenzaldéhyde par un ester acétoacétique de formule générale (V) :

$$H_3C-CO-CH_2-COOR_1 \quad (V)$$

dans laquelle $R_1$ a la même signification que dans la revendication 1, puis à traiter l'ester α-acétyl β-(2,3-méthylènedioxyphényl)acrylique résultant de formule (VI)

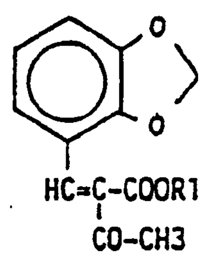

(VI)

dans laquelle $R_1$ conserve la même signification, par un ester 3-aminocrotonique de formule (VII)

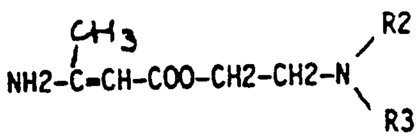

(VII)

dans laquelle $R_2$ et $R_3$ ont la même signification que dans la formule (III)

15. Un procédé de fabrication des composés de formule (III), selon la revendication 1, qui consiste à traiter le 2,3-méthylènedioxybenzaldéhyde par un ester acétoacétique de formule (VIII)

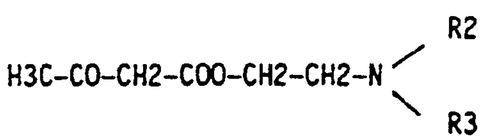

(VIII)

dans laquelle $R_2$ et $R_3$ ont la même signification que dans la formule (III), puis à traiter l'ester α-acétyl β-(2,3-méthylènedioxyphényl) acrylique résultant de formule (IX) :

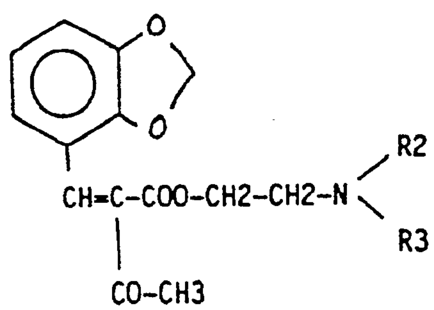

(IX)

dans laquelle $R_2$ et $R_3$ ont la même signification que dans la formule (III) par un ester 3-aminocrotonique de formule (X)

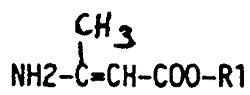

(X)

dans laquelle $R_1$ a la même signification que précédemment.

16. Un composé selon l'une quelconque des revendications 1 à 13, utilisé comme médicament pour traiter les troubles cardiovasculaires.

17. Une composition pharmaceutique comprenant un ou plusieurs composés selon l'une quelconque des revendications 1 à 13, associés avec un véhicule pharmaceutiquement acceptable.

**Revendications pour les pays contractants : AT, ES, GR**

1. Procédé de préparation de dérivés de 1,4-dihydropyridine de formule générale (III).

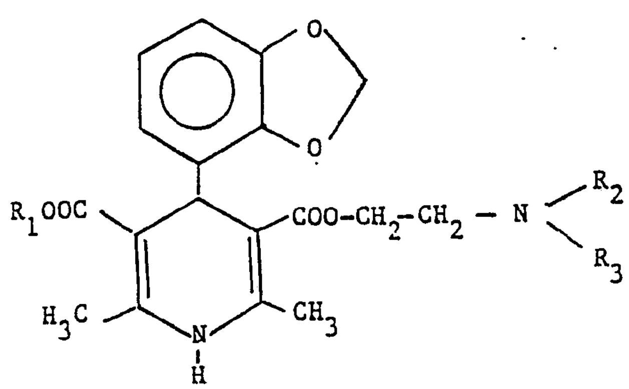

(III)

dans laquelle

- $R_1$ représente un groupe méthyle, éthyle, propyle, isopropyle, butyle ou isobutyle,
- $R_2$ représente un radical méthyle, formyle ou éthyle,
- $R_3$ représente un atome d'hydrogène ou un groupe 2-, 3-, ou 4-picolyle ou 4-fluorobenzyle, et de leurs sels d'addition avec un acide organique ou minéral, qui consiste à traiter le 2,3-méthylènedioxybenzaldéhyde par un ester acétoacétique de formule générale (V) :

$$H_3C\text{-}CO\text{-}CH2\text{-}COOR_1 \quad (V)$$

dans laquelle $R_1$ a la même signification que dans la formule (III), puis à traiter l'ester α-acétyl β-éthylènedioxyphényl)acrylique résultant de formule (VI) :

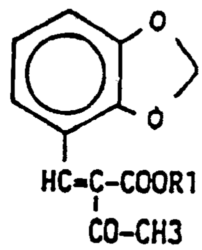

(VI)

dans laquelle $R_1$ conserve la même signification, par un ester 3-aminocrotonique de formule (VII) :

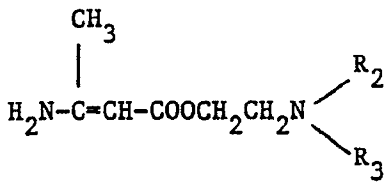

(VII)

dans laquelle $R_2$ et $R_3$ ont la même signification que dans la formule (III).

2. Un procédé de fabrication des composés de formule (III) telle que définie dans la revendication 1, qui consiste à traiter le 2,3-méthylènedioxy-benzaldéhyde par un ester acétoacétique de formule (VIII) :

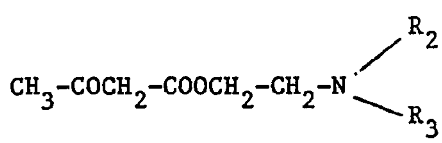

(VIII)

dans laquelle $R_2$ et $R_3$ ont la même signification que dans la formule (III), puis à traiter l'ester α-acétyl β-(2,3-méthylènedioxyphényl) acrylique résultant de formule (IX) :

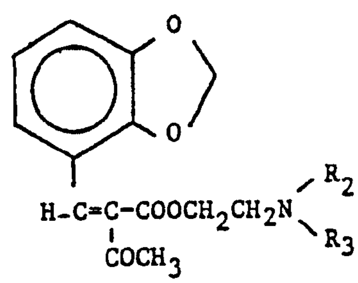

(IX)

dans laquelle $R_2$ et $R_3$ ont la même signification que dans la formule (III), par un ester 3-aminocrotonique de formule (X) :

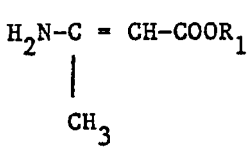

(X)

dans laquelle $R_1$ a la même signification que précédent.

3. Procédé de préparation de la 1,4-dihydro 2,6-diméthyl 4-(2',3'-méthylènedioxyphényl) 3-carboxylate de méthyle 5-carboxylate de 2-(N-formyl N-méthyl amino) éthyle pyridine, qui consiste à traiter le 2,3-méthylènedioxybenzaldéhyde par l'acéto-acétate de 2-(N-formyl N-méthylamino) éthyle puis à traiter l'α-acétyl β-(2,3-méthylènedioxy phényl) acrylate de 2-(N-formyl N-méthylamino) éthyle ainsi obtenu par le 3-amino crotonate de méthyle.

4. Procédé de préparation de la 1,4-dihydro 2,6-diméthyl 4-(2',3'-méthylènedioxyphényl) 3-carboxylate de méthyle 5-carboxylate de 2-(N-formyl N-benzylamino) éthyle pyridine, qui consiste à traiter le 2,3-méthylènedioxy benzaldéhyde par l'acétoacétate de 2-(N-formyl N-benzylamino)éthyle puis à traiter l'α-acétyl β-(2,3-méthylène dioxyphényl) acrylate de 2-(N-formyl N-benzylamino) éthyle ainsi obtenu par le 3-aminocrotonate de méthyle.

5. Procédé de préparation de la 1,4-dihydro 2,6-diméthyl 4-(2',3'-méthylènedioxyphényl) 3-carboxylate de méthyle 5-carboxylate de 2-(N-méthyl N-(2'-thiénylméthyl)amino)éthyle pyridine, qui consiste à traiter le 2,3-méthylène dioxy benzaldéhyde par l'acétoacétate de 2-(N-méthyl N-(2-thiénylméthyl)amino)éthyle puis à traiter l'α-acétyl β-(2,3-méthylènedioxyphényl) acrylate de 2-(N-méthyl N-(2'-thiénylméthyl) amino) éthyle ainsi obtenu par le 3-aminocrotonate de méthyle.

6. Procédé de préparation de la 1,4-dihydro 2,6-diméthyl 4-(2',3'-méthylènedioxyphényl) 3-carboxylate d'éthyle 5-carboxylate de 2-(N-méthyl N-(2'-thiénylméthyl) amino) éthyle pyridine, qui consiste à traiter le 2,3-méthylène dioxy benzaldéhyde par l'acétoacétate de 2-(N-méthyl N-(2'-thiénylméthyl)amino) éthyle puis à traiter l'α-acétyl β-(2,3-méthylènedioxyphényl) acrylate de 2-(N-méthyl N-(2'-thiénylméthyl) amino) éthyle ainsi obtenu par le 3-aminocrotonate d'éthyle.

7. Procédé de préparation de la 1,4-dihydro 2,6-diméthyl 4-(2',3'-méthylènedioxyphényl) 3-carboxylate d'isopropyle 5-carboxylate de 2-(N-méthyl N-(2'-thiénylméthyl)amino)éthyle pyridine, qui consiste à traiter le 2,3-méthylène dioxy benzaldéhyde par l'acétoacétate de 2-(N-méthyl N-(2'-thiénylméthyl) amino) éthyle puis à traiter l'α-acétyl β-(2,3-méthylènedioxy phényl) acrylate de 2-(N-méthyl N-(2'-thiényl méthyl) amino) éthyle ainsi obtenu par le 3-aminocrotonate d'isopropyle.

## Ansprüche

### Patentansprüche für die benannten Vertragsstaaten : DE, FR, GB, IT, LI, NL, SE

1. 1,4-Dihydropyridinderivate der allgemeinen Formel (III)

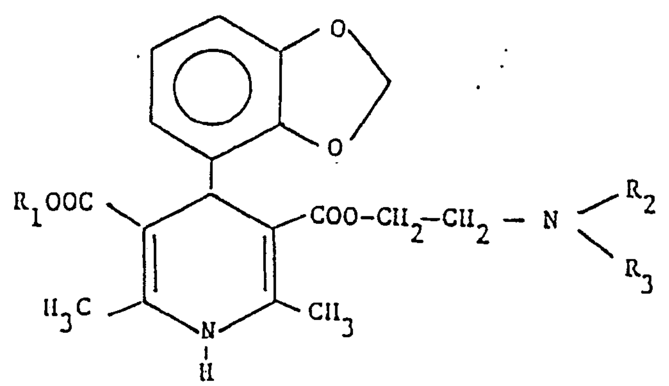

(III)

in der

$R_1$ eine Methyl-, Ethyl-, Propyl-, Isopropyl-, Butyl- oder Isobutylgruppe,

$R_2$ einen Methyl-, Formyl- oder Ethylrest und

$R_3$ ein Wasserstoffatom oder eine 2-, 3- oder 4-Picolyloder 4-Fluorbenzylgruppe

bedeuten, sowie ihre pharmazeutisch verträglichen Additionssalze mit einer Mineral- oder organischen Säure.

2. 1,4-Dihydro-2,6-dimethyl-4-(2',3'-methylendioxyphenyl)pyridin-3-carbonsäuremethylester-5-carbonsäure-2-(N-methylamino)-ethylester nach Anspruch 1.

3. 1,4-Dihydro-2,6-dimethyl-4-(2',3'-methylendioxyphenyl)pyridin-3-carbonsäuremethylester-5-carbonsäure-2-(N-formyl-N-methylamino)-ethylester.

4. 1,4-Dihydro-2,6-dimethyl-4-(2',3'-methylendioxyphenyl)pyridin-3-carbonsäuremethylester-5-carbonsäure-2-(N-formyl-N-benzylamino)-ethylester.

5. 1,4-Dihydro-2,6-dimethyl-4-(2',3'-methylendioxyphenyl)pyridin-3-carbonsäuremethylester-5-carbonsäure-2-(N-methyl-N-(2-picolyl)amino)-ethylester nach Anspruch 1.

6. 1,4-Dihydro-2,6-dimethyl-4-(2',3'-methylendioxyphenyl)pyridin-3-carbonsäuremethylester-5-carbonsäure-2-(N-methyl-N-(4-picolyl)amino)-ethylester nach Anspruch 1.

7. 1,4-Dihydro-2,6-dimethyl-4-(2',3'-methylendioxyphenyl)pyridin-3-carbonsäuremethylester-5-carbon-

säure-2-(N-methyl-N-(3-picolyl)amino)-ethylester nach Anspruch 1.

8. 1,4-Dihydro-2,6-dimethyl-4-(2′,3′-methylendioxyphenyl)pyridin-3-carbonsäuremethylester-5-carbonsäure-2-(N-methyl-N-(4′-fluorbenzyl)amino)-ethylester nach Anspruch 1.

9. 1,4-Dihydro-2,6-dimethyl-4-(2′,3′-methylendioxyphenyl)pyridin-3-carbonsäureethylester-5-carbonsäure-2-(N-methyl-N-(4′-fluorbenzyl)amino)-ethylester nach Anspruch 1.

10. 1,4-Dihydro-2,6-dimethyl-4-(2′,3′-methylendioxyphenyl)pyridin-3-carbonsäuremethylester-5-carbonsäure-2-(N-methyl-N-(2′-thienylmethyl)amino)-ethylester.

11. 1,4-Dihydro-2,6-dimethyl-4-(2′,3′-methylendioxyphenyl)pyridin-3-carbonsäureethylester-5-carbonsäure-2-(N-methyl-N-(2′-thienylmethyl)amino)ethylester.

12. 1,4-Dihydro-2,6-dimethyl-4-(2′,3′-methylendioxyphenyl)pyridin-3-carbonsäureisopropylester-5-carbonsäure-2-(N-methyl-N-(4′-fluorbenzyl)amino)-ethylester nach Anspruch 1.

13. 1,4-Dihydro-2,6-dimethyl-4-(2′,3′-methylendioxyphenyl)pyridin-3-carbonsäureisopropylester-5-carbonsäure-2-(N-methyl-N-(2′-thienylmethyl)amino)-ethylester.

14. Verfahren zur Herstellung von Verbindungen der Formel (III) gemäß Anspruch 1, dadurch gekennzeichnet, daß man 2,3-Methylendioxybenzaldehyd mit einem Acetessigester der allgemeinen Formel (V)

$$H_3C\text{-}CO\text{-}CH_2\text{-}COOR_1 \quad (V)$$

in der $R_1$ die gleiche Bedeutung wie im Anspruch 1 hat, behandelt und anschließend den erhaltenen alpha-Acetyl-beta-(2,3-methylendioxyphenyl)acrylester der Formel (VI)

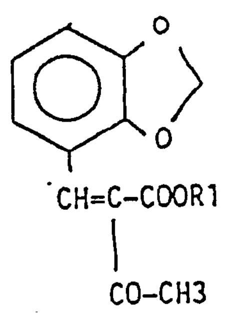

(VI)

in der $R_1$ die gleiche Bedeutung behält, mit einem 3-Aminocrotonester der Formel (VII)

$$NH_2\text{-}C(CH_3)\text{=}CH\text{-}COO\text{-}CH_2\text{-}CH_2\text{-}NR_2R_3 \quad (VII)$$

in der $R_2$ und $R_3$ die gleiche Bedeutung wie in Formel (III) haben, behandelt.

15. Verfahren zur Herstellung von Verbindungen der Formel (III) gemäß Anspruch 1, dadurch gekennzeichnet, daß man 2,3-Methylendioxybenzaldehyd mit einem Acetessigester der Formel (VIII)

$$H_3C\text{-}CO\text{-}CH_2\text{-}COO\text{-}CH_2\text{-}CH_2\text{-}NR_2R_3 \text{ (VIII)} \quad (VIII)$$

in der $R_2$ und $R_3$ die gleiche Bedeutung wie in Formel (III) haben, behandelt und anschließend den erhaltenen alpha-Acetyl-beta-(2,3-methylendioxyphenyl)-acrylester der Formel IX

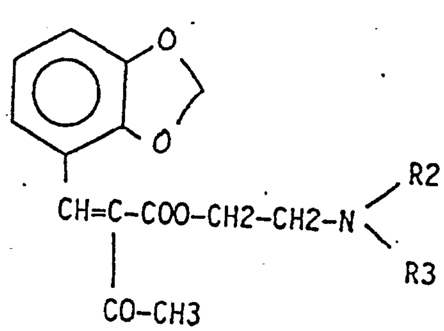

(IX)

in der $R_2$ und $R_3$ die gleiche Bedeutung wie in Formel (III) haben, mit einem 3-Aminocrotonester der Formel X

$$NH_2\text{-}C(CH_3)\text{=}CH\text{-}COO\text{-}R_1 \quad (X)$$

in der $R_1$ wie oben definiert ist, behandelt.

16. Verbindung nach einem der Ansprüche 1 bis 13 zur Verwendung als Arzneimittel für die Behandlung cardiovasculärer Störungen.

17. Pharmazeutische Zusammensetzung, enthaltend eine Verbindung oder mehrere gemäß einem der Ansprüche 1 bis 13 zusammen mit einem pharmazeutisch verträglichen Träger.

**Patentansprüche für die benannten Vertragsstaaten : AT, ES, GR**

1. Verfahren zur Herstellung von 1,4-Dihydropyridinderivaten der allgemeinen Formel (III)

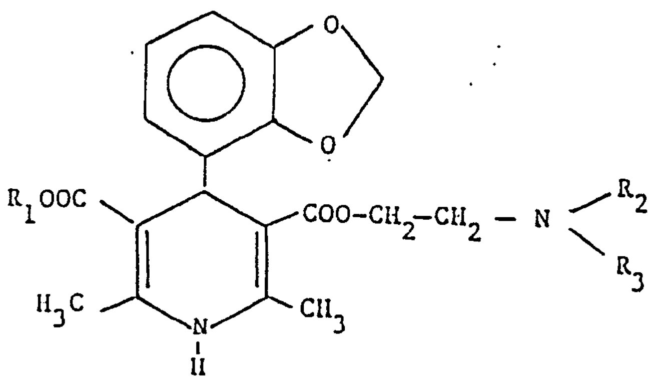

(III)

in der

$R_1$ eine Methyl-, Ethyl-, Propyl-, Isopropyl-, Butyl- oder Isobutylgruppe,

$R_2$ einen Methyl-, Formyl- oder Ethylrest und

$R_3$ ein Wasserstoffatom oder eine 2-, 3- oder 4-Picolyl-oder 4-Fluorbenzylgruppe

bedeuten, sowie ihrer pharmazeutisch verträglichen Additionssalze mit Mineral- oder organischen Säuren, dadurch gekennzeichnet, daß man 2,3-Methylendioxybenzaldehyd mit einem Acetessigester der allgemeinen Formel (V)

$$H_3C\text{-}CO\text{-}CH_2\text{-}COOR_1 \quad (V)$$

in der $R_1$ die gleiche Bedeutung wie in Formel (III) hat, behandelt und anschließend den erhaltenen alpha-Acetyl-beta-(2,3-methylendioxyphenyl)acrylester der Formel (VI)

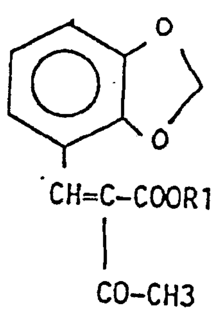

(VI)

in der $R_1$ wie oben definiert ist, mit einem 3-Aminocrotonester der Formel (VII)

$$NH_2\text{-}C(CH_3)\text{=}CH\text{-}COO\text{-}CH_2\text{-}CH_2\text{-}NR_3R_3 \quad (VII)$$

in der $R_2$ und $R_3$ die gleiche Bedeutung wie in Formel (III) haben, behandelt.

2. Verfahren zur Herstellung von Verbindungen der Formel (III) gemäß Anspruch 1, dadurch gekennzeichnet, daß man 2,3-Methylendioxybenzaldehyd mit einem Acetessigester der Formel (VIII)

$$H_3C\text{-}CO\text{-}CH_2\text{-}COO\text{-}CH_2\text{-}CH_2\text{-}NR_2R_3 \quad (VIII)$$

in der $R_2$ und $R_3$ die gleiche Bedeutung wie in Formel (III) haben, behandelt und anschließend den erhaltenen alpha-Acetyl-beta-(2,3-methylendioxyphenyl)-acrylester der Formel (IX)

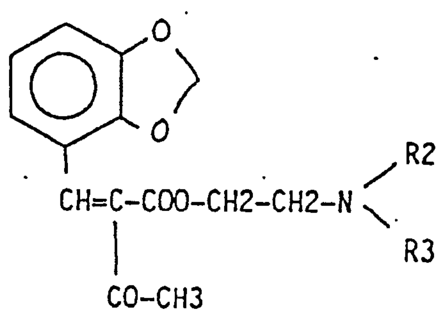

(IX)

in der $R_2$ und $R_3$ die gleiche Bedeutung wie in Formel III haben, mit einem 3-Aminocrotonester der Formel X

$$NH_2\text{-}C(CH_3)\text{=}CH\text{-}COO\text{-}R_1 \quad (X)$$

in der $R_1$ wie oben definiert ist, behandelt.

3. Verfahren zur Herstellung von 1,4-Dihydro-2,6-dimethyl-4-(2',3'-methylendioxyphenyl)-pyridin-3-carbonsäuremethylester-5-carbonsäure-2-(N-formyl-N-methylamino)-ethylester, dadurch gekennzeichnet, daß man 2,3-Methylendioxybenzaldehyd mit 2-(N-Formyl-N-methylamino)ethyl-acetylacetat behandelt und anschließend das erhaltene 2-(N-Formyl-N-methylamino)ethyl-alpha-acetyl-beta-(2',3'-methylendioxyphenyl)acrylat mit 3-Aminocrotonsäuremethylester behandelt.

4. Verfahren zur Herstellung von 1,4-Dihydro-2,6-dimethyl-4-(2',3'-methylendioxyphenyl)-pyridin-3-carbonsäuremethylester-5-carbonsäure-2-(N-formyl-N-benzylamino)-ethylester, dadurch gekennzeichnet, daß man 2,3-Methylendioxybenzaldehyd mit 2-(N-Formyl-N-benzylamino)ethyl-acetylacetat behandelt und anschließend das erhaltene 2-(N-Formyl-Nbenzylamino)ethyl-alpha-acetyl-beta-(2',3'-methylendioxyphenyl)acrylat mit 3-Aminocrotonsäuremethylester behandelt.

5. Verfahren zur Herstellung von 1,4-Dihydro-2,6-dimethyl-4-(2',3'-methylendioxyphenyl)-pyridin-3-carbonsäuremethylester-5-carbonsäure-2-(N-methyl-N-(2'-thienylmethyl)amino)-ethylester, dadurch gekennzeichnet, daß man 2,3-Methylendioxybenzaldehyd mit 2-(N-Methyl-N-(2'-thienylmethyl)amino)ethyl-acetylacetat behandelt und anschließend das erhaltene 2-(N-Methyl-N-(2'-thienyl)methyl-amino)ethyl-alpha-acetyl-beta-(2',3'-methylendioxyphenyl)acrylat mit 3-Aminocrotonsäuremethylester behandelt.

6. Verfahren zur Herstellung von 1,4-Dihydro-2,6-dimethyl-4(2',3'-methylendioxyphenyl)-pyridin-3-carbonsäureethylester-5-carbonsäure-2-(N-methyl-N-(2'-thienylmethyl)amino)-ethylester, dadurch gekennzeichnet, daß man 2,3-Methylendioxybenzaldehyd mit 2-(N-Methyl-N-(2'-thienyl)methyl-amino)ethyl-acetylacetat behandelt und anschließend das erhaltene 2-(N-Methyl-N-(2'-thienylmethyl)amino)ethyl-alpha-acetyl-beta-(2',3'-methylendioxyphenyl)acrylat mit 3-Aminocrotonsäureethylester behandelt.

7. Verfahren zur Herstellung von 1,4-Dihydro-2,6-dimethyl-4-(2',3'-methylendioxyphenyl)-pyridin-3-carbonsäureisopropylester-5-carbonsäure-2-(N-methyl-N-(2'-thienylmethyl)amino)-ethylester, dadurch gekennzeichnet, daß man 2,3-Methylendioxybenzaldehyd mit 2-(N-Methyl-N-(2'-thienylmethyl)-amino)ethyl-acetylacetat behandelt und anschließend das erhaltene 2-(N-Methyl-N-(2'-thienylmethyl)-amino)ethyl-alpha-acetyl-beta(2',3'-methylendioxyphenyl)acrylat mit 3-Aminocrotonsäureisopropylester behandelt.

## Claims

### Claims for the contracting states : DE, FR, GB, IT, LI, NL, SE

1. 1,4-dihydropyridine derivatives of the general formula (III)

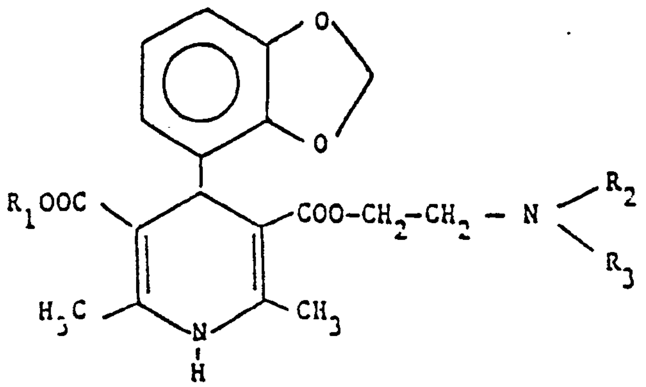

(III)

wherein :

- $R_1$ represents a methyl, ethyl, propyl, isopropyl, butyl or isobutyl group,
- $R_2$ represents a methyl, formyl or ethyl radical,
- $R_3$ represents a hydrogen atom, or a 2-, 3-, or 4-picolyl or 4-fluorobenzyl group,

and pharmaceutically acceptable additive salts thereof, with an inorganic or organic acid.

2. According to Claim 1, 3-methyl 5-[2-(N-methylamino)ethyl] 1,4-dihydro-2,6-dimethyl-4-(2,3-methylenedioxyphenyl)-3,5-pyridinedicarboxylate

3. 3-Methyl 5-[2-(N-formyl-N-methyl(amino)ethyl] 1,4-dihydro-2,6-dimethyl-4-(2,3-methylenedioxyphenyl)-3,5-pyridinedicarboxylate.

4. 3-Methyl 5-[2-(N-formyl-N-benzylamino)ethyl] 1,4-dihydro-2,6-dimethyl-4-(2′,3′-methylenedioxyphenyl)-3,5-pyridinedicarboxylate.

5. According to Claim 1, 3-Methyl 5-[(2-(N-methyl-N-(2-picolyl)amino)ethyl] 1,4-dihydro-2,6-dimethyl-4-(2,3-methylenedioxyphenyl)-3,5-pyridinedicarboxylate.

6. According to Claim 1, 3-Methyl 5-[(2-(N-methyl-N-(4-picolyl)amino)ethyl] 1,4-dihydro-2,6-dimethyl-4-(2,3-methylenedioxyphenyl)-3,5-pyridinedicarboxylate.

7. According to Claim 1, 3-Methyl 5-[(2-(N-methyl-N-(3-picolyl)amino)ethyl] 1,4-dihydro-2,6-dimethyl-4-(2′,3′-methylene dioxyphenyl)-3,5-pyridinedicarboxylate.

8. According to Claim 1, 3-methyl 5-[2-(N-methyl-N-(4-fluorobenzyl)amino)ethyl] 1,4-dihydro-2,6-dimethyl-4-(2′,3′-methylene dioxyphenyl)-3,5-pyridinedicarboxylate.

9. According to, Claim 1, 3-ethyl 5-[(2-(N-methyl-N-(4-fluorobenzyl)amino)ethyl] 1,4-dihydro-2,6-dimethyl-4-(2′,3′-methylene dioxyphenyl)-3,5-pyridine dicarboxylate.

10. 3-Methyl 5-[2-(N-methyl-N-(2-thienyl-methyl)amino)ethyl] 1,4-dihydro-2,6-dimethyl-4-(2′,3′-methylenedioxyphenyl)-3,5-pyridinedicarboxylate

11. 3-Ethyl 5-[2-(N-methyl-N-(2′-thienylmethyl)amino)ethyl] 1,4dihydro-2,6-dimethyl-4-(2′,3′-methylenedioxyphenyl)-3,5-pyridinedicarboxylate.

12. According to Claim 1, 3-isopropyl 5-[2-(N-methyl-N-(4-fluorobenzyl)amino)ethyl] 1,4-dihydro-2,6-dimethyl-4-(2′,3′-methylenedioxy phenyl)-3,5-pyridine dicarboxylate.

13. 3-isopropyl 5-[2-(N-methyl-N-(2-thienylmethyl)amino)ethyl] 1,4-dihydro-2,6-dimethyl-4-(2,3-methylenedioxyphenyl)-3,5-pyridine dicarboxylate.

14. A process for preparing a compound of Formula (III) according to Claim 1, comprising treating 2,3-methylenedioxybenzaldehyde with an acetoacetic ester of general Formula (V) :

$$H_3C\text{-}CO\text{-}CH_2\text{-}COOR_1 \quad (V)$$

in which $R_1$ is as defined in Claim 1, and then treating the resulting α-acetyl-β-(2,3-methylene-dioxyphenyl)acrylic ester of Formula (VI)

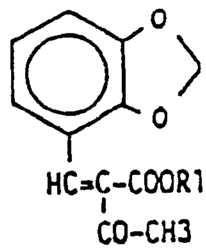

(VI)

in which $R_1$ has the same meaning as in Claim 1, with a 3-amino-crotonic ester of Formula (VII)

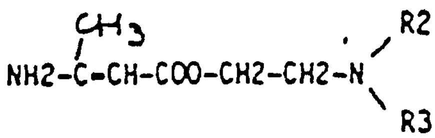

(VII)

in which $R_2$ and $R_3$ are as defined in formula III.

15. A process for the production of compounds of formula (III) according to Claim 1, which consists in treating 2,3-methylenedioxybenzaldehyde with an acetoacetic ester of formula (VIII)

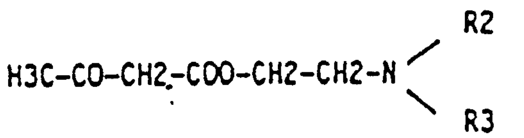

(VIII)

in which $R_2$ and $R_3$ have the same meaning as in formula (III), then in treating the resulting α-acetyl-β-(2,3-methylenedioxyphenyl) acrylic ester of formula (IX) :

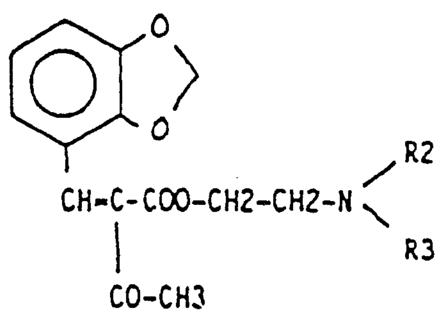

(IX)

in which $R_2$ and $R_3$ have the same meaning as in formula (III) with a 3-aminocrotonic ester of formula (X)

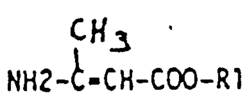

(X)

in which $R_1$ has the same meaning as before

16. A compound according to any one of Claims 1-13, used as a medicine for treating cardiovascular disorders.

17. A pharmaceutical composition consisting of one or a plurality of compounds according to any of Claims 1 to 13, associated with a pharmaceutically acceptable vehicle.

**Claims for the contracting states : AT, ES, GR**

1. A process for the preparation of 1,4-dihydropyridine derivatives of general formula (III).

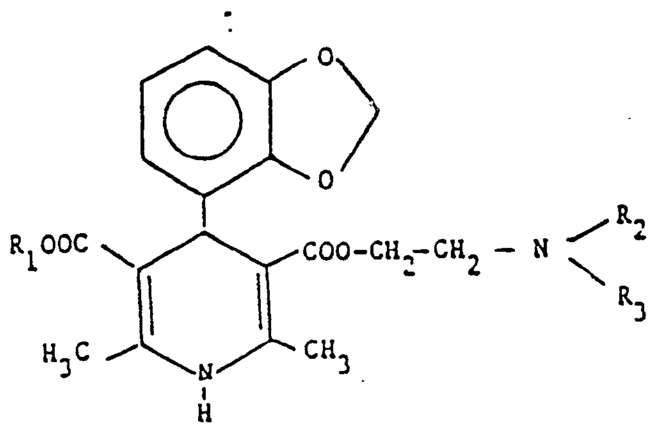

(III)

in which

- $R_1$ represents a methyl, ethyl, propyl, isopropyl, butyl or isobutyl group,
- $R_2$ represents a methyl, formyl or ethyl radical,
- $R_3$ represents a hydrogen atom or a 2-, 3-, or 4-picolyl or 4-fluorobenzyl group,

and addition salts thereof with an organic or inorganic acid, which involves treating the 2,3-methylenedioxybenzaldehyde with an acetoacetic ester of general formula (V) :

$$H_3C\text{-}CO\text{-}CH_2\text{-}COOR_1 \quad (V)$$

in which $R_1$ has the same meaning as in Formula (III), then in treating the resulting α-acetyl β-(2,3-methylenedioxyphenyl)acrylic ester of formula (VI) :

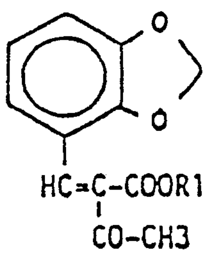

(VI)

(in which $R_1$ has the same meaning as above) with a 3-aminocrotonic ester of formula (VII) :

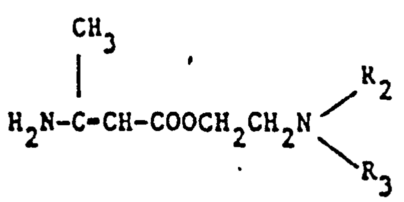

(VII)

in which $R_2$ and $R_3$ have the same meaning as in formula (III).

2. A process for the manufacture of compounds of formula (III) such as defined in Claim 1, which consists in treating the 2,3-methylenedioxybenzaldehyde with an acetoacetic ester of formula (VIII) :

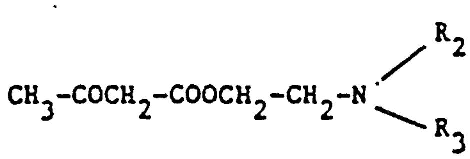

(VIII)

in which $R_2$ and $R_3$ have the same meaning as in Formula (III), then in treating the resulting α-acetyl β-(2,3-methylenedioxyphenyl) acrylic ester of formula (IX) :

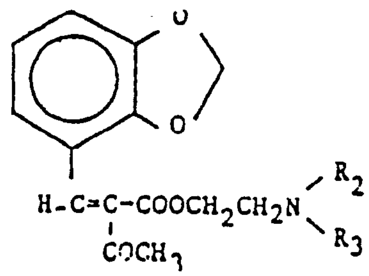

(IX)

in which $R_2$ and $R_3$ have the same meaning as in Formula (III), with a 3-aminocrotonic ester of formula (X) :

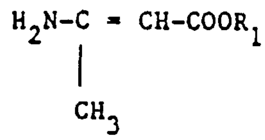

in which $R_1$ has the same meaning as before.

3. A process for the preparation of 3-Methyl 5-[2'-(N-formyl-N-methylamino)ethyl] 1,4-dihydro-2,6-dimethyl-4-(2',3'-methylenedioxyphenyl)-3,5-pyridinedicarboxylate, which consists in treating the 2,3-methylenedioxybenzaldehyde with 2-(N-formyl N-methylamino) ethyl acetylacetate, and then in treating the 2-(N-Formyl-N-methylamino)ethyl α-acetyl-β-(2,3-methylenedioxyphenyl) acrylate thus obtained with methyl 3-aminocrotonate.

4. A process for the preparation of 3-Methyl 5-[2-(N-formyl-N-benzylamino)ethyl] 1,4-dihydro-2,6-dimethyl-4-(2',3'-methylenedioxyphenyl)-3,5-pyridine-dicarboxylate, which consists in treating 2,3-methylenedioxybenzaldehyde with 2-(N-formyl-N-benzylamino)ethyl acetoacetate, and then in treating 2-(N-formyl-N-benzylamino)ethyl α-acetyl-β-(2,3-methylenedioxyphenyl)acrylate thus obtained with methyl 3-aminocrotonate.

5. A process for the preparation of 3-Methyl 5-{2-[N-methyl-N-(2-thienylmethyl)amino]ethyl} 1,4-dihydro-2,6-dimethyl-4-(2,3-methylene dioxyphenyl)-3,5-pyridinedicarboxylate, which consists in treating the 2,3-methylenedioxybenzaldehyde with 2-(N-methyl N-(2'-thienylmethyl)amino) ethyl acetoacetate, then in treating 2-(N-methyl N-(2'-thienylmethyl)amino)ethyl α-acetyl-β-(2,3-methylenedioxyphenyl)acrylate thus obtained with methyl 3-aminocrotonate.

6. A process for the preparation of 3-ethyl 5-{2-[N-methyl-N-(2-thienylmethyl)amino]ethyl} 1,4-dihydro-2,6-dimethyl-4-(2,3-methylene dioxyphenyl)-3,5-pyridinedicarboxylate, which consists in treating the 2,3-methylene dioxy benzaldehyde with 2-(N-methyl N-(2'-thienylmethyl)amino) ethylacetoacetate, then intreating the 2-(N-methyl-N-(2'-thienylmethyl)amino) ethyl α-acetyl β-(2,3-methylenedioxyphenyl) acrylate thus obtained with ethyl 3-aminocrotonate.

7. A process for the preparation of 3-isopropyl 5-{2-[N-methyl-N-(2-thienylmethyl)amino]ethyl} 1,4-dihydro-2,6-dimethyl-4-(2,3-methylene dioxyphenyl)-3,5-pyridinedicarboxylate, which consists in treating the 2,3-methylene dioxy benzaldehyde with 2-(N-methyl N-(2'-thienylmethyl)amino) ethyl acetoacetate, then in treating the 2-(N-methyl N-(2'-thienyl methyl)amino)ethyl α-acetyl β-(2,3-methylnedioxyphenyl) acrylate thus obtained with the isopropyl 3-aminocrotonate.